(19) Europäisches Patentamt
European Patent Office
Office européen des brevets

(11) Veröffentlichungsnummer: **0 300 341**
**A2**

# (12) EUROPÄISCHE PATENTANMELDUNG

(21) Anmeldenummer: **88111181.9**

(22) Anmeldetag: **13.07.88**

(51) Int. Cl.4: **C07D 243/24 , C07B 59/00 , G01N 33/534**

(30) Priorität: **24.07.87 US 77472**
**06.11.87 US 117599**

(43) Veröffentlichungstag der Anmeldung: **25.01.89 Patentblatt 89/04**

(84) Benannte Vertragsstaaten: **BE CH DE FR GB IT LI NL**

(71) Anmelder: **F. HOFFMANN-LA ROCHE & CO. Aktiengesellschaft**

**CH-4002 Basel(CH)**

(72) Erfinder: **Earley, James Valentine**
**40 Daniel Drive**
**Cedar Grove, N.J. 07009(US)**
Erfinder: **Gilman, Norman Washburn**
**5 Normandy Drive**
**Wayne, N.J. 07470(US)**

(74) Vertreter: **Kloter, Rudolf et al**
**Postfach 3255 Grenzacherstrasse 124**
**CH-4002 Basel(CH)**

(54) **Neue Benzodiazepin-Derivate, radioaktiv markierte Derivate davon sowie die Verwendung der letzteren in Immunverfahren zum Nachweis von Benzodiazepinen.**

(57) Es werden Benzodiazepine der Formel

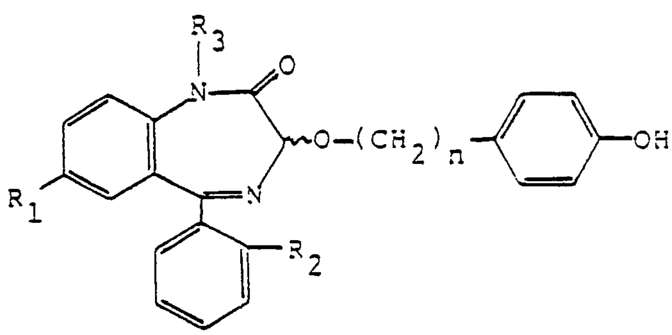

I

beschrieben, worin $R_1$ Halogen oder Nitro bedeutet, $R_2$ Wasserstoff oder Halogen bedeutet, $R_3$ Wasserstoff oder niederes Alkyl bedeutet und n 2-6 ist. Diese Verbindungen sind in radioaktiv markierter Form, besonders mit $J^{125}$ markiert, wertvoll in einem Immunverfahren zur Bestimmung der Gegenwart von Benzodiazepin.

EP 0 300 341 A2

# Neue Benzodiazepin-Derivate, radioaktiv markierte Derivate davon sowie die Verwendung der letzteren in Immunverfahren zum Nachweis von Benzodiazepinen

Publizierte Uebersichten zeigen, dass nicht weniger als einer von zehn Erwachsenen Amerikanern innerhalb von Jahresfrist einen Benzodiazepin-Tranquilizer oder ein Benzodiazepin-Hypnotikum zu sich nimmt. Diese Verbindungen werden bei zahlreichen Zuständen verschrieben, z.B. bei neurotischer Aengstlichkeit, Depressionen, Schlaflosigkeit und Muskelspasmen sowie bei zahllosen funktionellen Fehlzuständen, die von Kopfschmerzen bis zu Dyspareunien reichen (Benzodiazepins in Clinical Medicine, Greenblatt and Shader, Raven Press, 1974).

Nach der Einnahme von therapeutischen Dosen von Benzodiazepinen sind, bedingt durch die extensive Biotransformation und die Gewebeverteilung, nur Spuren in Körperflüssigkeiten vorhanden. Aus diesem Grunde sind sehr sensitive Methoden für die Quantifizierung von Benzodiazepinen in Gewebe und Körperflüssigkeiten notwendig.

Es existiert eine Vielzahl von Suchmethoden für die Detektion von Benzodiazepinen in Blut, Urin oder Körperflüssigkeiten. Hierzu wurden Dünnschichtchromatographie oder Gasflüssigchromatographie oder Spectrophotofluorometrie verwendet. In neuerer Zeit wurden immunologische Methoden entwickelt, um mit Hilfe von Antikörpern gegen Benzodiazepine kleine Mengen von Benzodiazepinen in Körperflüssigkeiten erfolgreich zu quantifizieren. Bei dieser Technologie ist es besonders notwendig, eine Verbindung zu haben, welche als Substrat wirkt und welche an den "Tracer" (welcher die Bestimmung und Quantifizierung des unbekannten Analyten erlaubt) binden kann. Zahlreiche Benzodiazepinderivate wurden für diesen Zweck verwendet. In diesem Zusammenhang sei auch die U.S.-Patentschrift Nr. 4,083,948 von Davis et al. hingewiesen, in welcher ein Benzodiazepin Radioimmunverfahren beschrieben ist, bei den $J^{125}$ markierte Benzodiazepinderivate als Substrate verwendet werden.

Die vorliegende Erfindung umfasst neue Benzodiazepinderivate. Die vorliegende Erfindung umfasst auch radioaktiv markierte neue Benzodiazepinderivate.

Weiterhin umfasst die vorliegende Erfindung die Verwendung der Verbindungen gemäss vorliegender Erfindung in Immunassays zum Auffinden des Vorliegens von Benzodiazepin.

Die vorliegende Erfindung umfasst Verbindungen der Formel

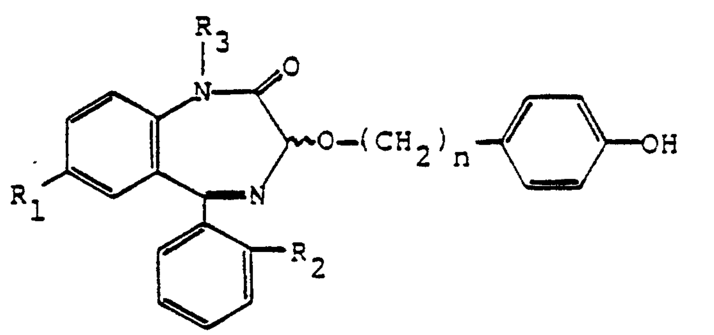

I

worin $R_1$ Halogen oder Nitro, $R_2$ Wasserstoff oder Halogen und $R_3$ Wasserstoff oder niederes Alkyl und n 2-6 ist.

Die vorliegende Erfindung umfasst ebenfalls die Verbindungen der obigen Formel, welche radioaktiv sind. Die Verbindungen der Formel I sind sehr leicht radioaktiv zu markieren. Das radioaktive Molekül wird entweder an die 3-Stellung oder die 3- und 5-Stellung des Hydroxy phenylrests der Verbindung der Formel I gebunden. Bevorzugt sind die Verbindungen mit $J^{125}$ markiert.

Besonders bevorzugt ist eine Verbindung der Formel I worin $R_1$ Chlor, $R_2$ Wasserstoff, $R_3$ Wasserstoff und n 2 ist, wobei diese Verbindung die Formel

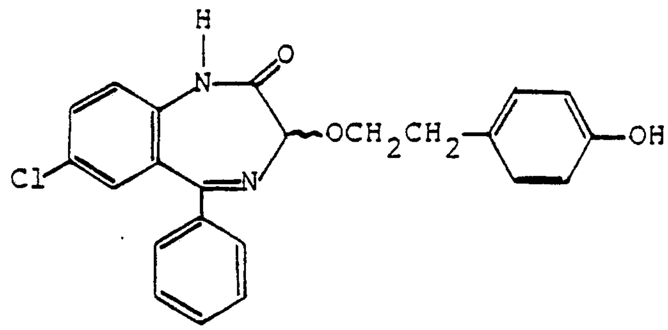

I(a)

aufweist.

Ebenfalls besonders bevorzugt ist eine Verbindung der Formal I(a), welche radioaktiv markiert ist. Das radioaktive Molekül bindet an die 3- oder die 3- und 5-Stellung des Hydroxyphenylrests der Verbindung der Formel I(a). Besonders bevorzugt ist eine Verbindung der Formel I(a), welche mit $J^{125}$ markiert ist und welche Verbindung $J^{125}$-7-Chlor-3-[2-(4-hydroxyphenyl)äthoxy]-5-phenyl-1,3-dihydro-2H-1,4-benzodiazepin-2-on ist.

Die Zwischenprodukte die zur Herstellung der Verbindungen der Formel I verwendet werden, sind bekannt und entsprechen der allgemeinen Formel

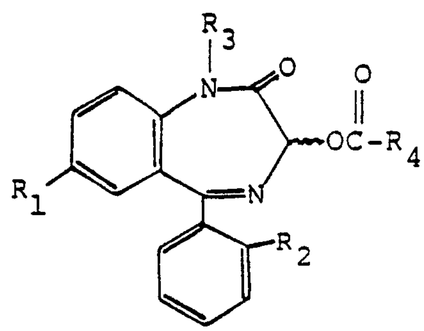

II

worin $R_1$, $R_2$ und $R_3$ wie obenerwähnt definiert sind und worin $R_4$ niederes Alkyl oder halogensubstituiertes niederes Alkyl ist.

Der Begriff niederes Alkyl bedeutet geradkettiges oder verzweigtes Alkyl mit bis zu 6 C-Atomen, vorzugsweise bis zu 3 C-Atomen.

Bevorzugt ist das Zwischenprodukt der Formel II, worin $R_1$ Chlor, $R_2$ Wasserstoff, $R_3$ Wasserstoff und $R_4$ Methyl ist, wobei die Verbindung die Formel

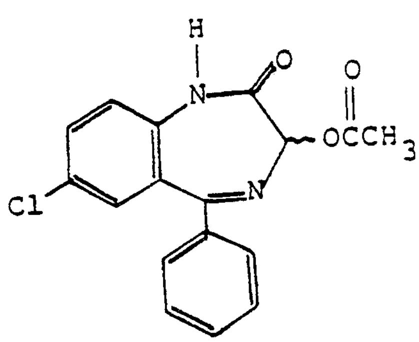

II(a)

aufweist.

Die Verbindungen der Formel I können hergestellt werden durch Reaktion einer Verbindung der Formel II mit einem 4-Hydroxyphen$(CH_2)_n$ Alkohol in trockenem N,N-Dimethylformamid. Die Lösung wird kalt gehalten und gerührt, wobei Chlorwasserstoffgas in die Lösung geleitet wird. Das Reaktionsgemisch wird dann bei Raumtemperatur gehalten und erneut gekühlt, worauf der pH mit einer Base auf 7,1 bis 7,9 adjustiert wird. Das Gemisch wird dann filtriert und der gummi-ähnliche Niederschlag wird mit Dichlormethan und Wasser gerührt und dann filtriert. Der Feststoff wird aus einer Mischung von Dichlormethan und Methanol kristallisiert und liefert die Verbindung der Formel I.

Die Erfindung umfasst weiterhin die Verwendung der radioaktiv markierten Verbindung der Formel I in einem Immunassay zum Auffinden der Gegenwart von Benzodiazepinen.

Besonders bevorzugt ist die Verbindung der Formel I, wenn sie mit $J^{125}$ radioaktiv markiert ist. Eine besonders bevorzugte $J^{125}$ markierte Verbindung der Formel I ist $J^{125}$-7-Chlor-3-[2-(4-hydroxyphenyl)-äthoxy]-5-phenyl-1,3-dihydro-2H-1,4-benzodiazepin-2-on.

Das bevorzugte Radioimmunverfahren zur Bestimmung der Benzodiazepinen basiert auf dem Kompetitionsprinzip. Die Verbindungen der Formel I werden zuerst nach bekannten Methoden radioaktiv markiert. Bevorzugt ist, wenn die Verbindungen der Formel I mit $J^{125}$ radioaktiv markiert werden, und zwar nach der in der U.S.-Patentschrift Nr. 4,083,948 beschriebenen Methode, um $J^{125}$ markierte Verbindungen zu erhalten. Besonders bevorzugt ist, wenn die radioaktiv markierte Verbindung der Formel I $J^{125}$-7-Chlor-3-[2-(4-hydroxyphenyl)äthoxy]-5-phenyl-1,3-dihydro-2H-1,4-benzodiazepin-2-on in einem Radioimmunverfahren zur Bestimmung von Benzodiazepinen verwendet wird.

Die radioaktiv markierten Verbindungen der Formel I binden an Antikörper gegen Benzodiazepine. Eine unbekannte Probe enthaltend eine unbekannte Menge von Benzodiazepinen oder von deren Metaboliten wird in das System eingesetzt. Allfällige vorhandene Benzodiazepine in der unbekannten Probe konkurrieren mit den jodinierten Verbindungen der Formel I um die Antikörper und erlauben die Quantifizierung der Menge an Benzodiazepinen in der unbekannten Probe. Die radioaktiv markierten Verbindungen der Formel I werden dann in einem Radioimmunverfahren zur Bestimmung der Gegenwart von Benzodiazepinen verwendet. Die Methodik dieser Radioimmunverfahren ist bekannt und in der U.S.-Patentschrift Nr. 4,083,948 beschrieben.

Die vorliegende Erfindung wird weiterhin im Zusammenhang mit den folgenden Beispielen beschrieben, welche jedoch lediglich zur Illustration dienen.

## Beispiel 1

### Herstellung von 7-Chlor-3-[2-(4-hydroxyphenyl)ethoxy]-5-phenyl-1,3-dihydro-2H-1,4-benzodiazepin-2-on

Eine Lösung von 10,0 g (0,0309 mol) 3-Acetoxy-7-chlor-5-phenyl-1,3-dihydro-2H-1,4-benzodiazepin-2-on und 11,0 g (0,080 mol) 4-Hydroxyphenothylalkohol (Aldrich) in 50 ml trockenem N,N-Dimethylformamid wird in Eisbad gerührt, wobei während 5 bis 10 Min. Chlorwasserstoffgas in die Lösung eingeleitet wird. Nach Stehenlassen des Reaktionsgemisches während 18 Std. bei Raumtemperatur wird das Reaktionsgemisch auf Eis gegossen und der pH wird mit Ammoniumhydroxid auf 7,5 eingestellt. Die Mischung wird filtriert und der gummiähnliche Rückstand wird während 5 Min. mit 50 ml Dichlormethan und 50 ml Wasser gewaschen und dann filtriert. Der Feststoff wird aus einer Mischung von Dichlormethan und Methanol kristallisiert und liefert 9,6 g (77%) der Verbindung der Formel I(a) als weisse Stäbchen vom Schmelzpunkt 222-230 °C.

than und Wasser gerührt und dann filtriert. Der Feststoff wird aus einer Mischung von Dichlormethan und Methanol kristallisiert und liefert die Verbindung der Formel I.

Die Erfindung umfasst weiterhin die Verwendung der radioaktiv markierten Verbindung der Formel I in einem Immunassay zum Auffinden der Gegenwart von Benzodiazepinen.

Besonders bevorzugt ist die Verbindung der Formel I, wenn sie mit $J^{125}$ radioaktiv markiert ist. Eine besonders bevorzugte $J^{125}$ markierte Verbindung der Formel I ist $J^{125}$-7-Chlor-3-[2-(4-hydroxyphenyl)-äthoxy]-5-phenyl-1,3-dihydro-2H-1,4-benzodiazepin-2-on.

Das bevorzugte Radioimmunverfahren zur Bestimmung der Benzodiazepinen basiert auf dem Kompetitionsprinzip. Die Verbindungen der Formel I werden zuerst nach bekannten Methoden radioaktiv markiert. Bevorzugt ist, wenn die Verbindungen der Formel I mit $J^{125}$ radioaktiv markiert werden, und zwar nach der in der U.S.-Patentschrift Nr. 4,083,948 beschriebenen Methode, um $J^{125}$ markierte Verbindungen zu erhalten. Besonders bevorzugt ist, wenn die radioaktiv markierte Verbindung der Formel I $J^{125}$-7-Chlor-3-[2-(4-hydroxyphenyl)äthoxy]-5-phenyl-1,3-dihydro-2H-1,4-benzodiazepin-2-on in einem Radioimmunverfahren zur Bestimmung von Benzodiazepinen verwendet wird.

Die radioaktiv markierten Verbindungen der Formel I binden an Antikörper gegen Benzodiazepine. Eine unbekannte Probe enthaltend eine unbekannte Menge von Benzodiazepinen oder von deren Metaboliten wird in das System eingesetzt. Allfällige vorhandene Benzodiazepine in der unbekannten Probe konkurrieren mit den jodinierten Verbindungen der Formel I um die Antikörper und erlauben die Quantifizierung der Menge an Benzodiazepinen in der unbekannten Probe. Die radioaktiv markierten Verbindungen der Formel I werden dann in einem Radioimmunverfahren zur Bestimmung der Gegenwart von Benzodiazepinen verwendet. Die Methodik dieser Radioimmunverfahren ist bekannt und in der U.S.-Patentschrift Nr. 4,083,948 beschrieben.

Die vorliegende Erfindung wird weiterhin im Zusammenhang mit den folgenden Beispielen beschrieben, welche jedoch lediglich zur Illustration dienen.

## Beispiel 1

### Herstellung von 7-Chlor-3-[2-(4-hydroxyphenyl)ethoxy]-5-phenyl-1,3-dihydro-2H-1,4-benzodiazepin-2-on

Eine Lösung von 10,0 g (0,0309 mol) 3-Acetoxy-7-chlor-5-phenyl-1,3-dihydro-2H-1,4-benzodiazepin-2-on und 11,0 g (0,080 mol) 4-Hydroxyphenothylalkohol (Aldrich) in 50 ml trockenem N,N-Dimethylformamid wird in Eisbad gerührt, wobei während 5 bis 10 Min. Chlorwasserstoffgas in die Lösung eingeleitet wird. Nach Stehenlassen des Reaktionsgemisches während 18 Std. bei Raumtemperatur wird das Reaktionsgemisch auf Eis gegossen und der pH wird mit Ammoniumhydroxid auf 7,5 eingestellt. Die Mischung wird filtriert und der gummiähnliche Rückstand wird während 5 Min. mit 50 ml Dichlormethan und 50 ml Wasser gewaschen und dann filtriert. Der Feststoff wird aus einer Mischung von Dichlormethan und Methanol kristallisiert und liefert 9,6 g (77%) der Verbindung der Formel I(a) als weisse Stäbchen vom Schmelzpunkt 222-230 °C.

**Ansprüche**

1. Eine Verbindung der Formel

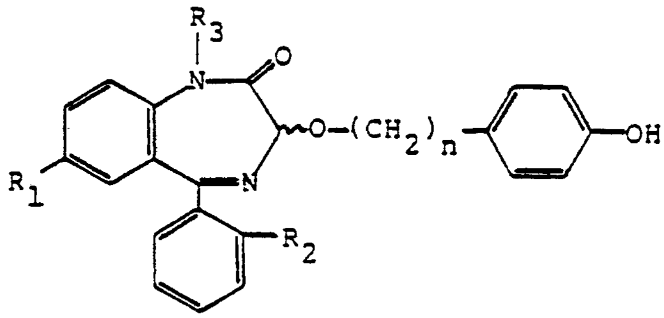

I

worin $R_1$ Halogen oder Nitro bedeutet, $R_2$ Wasserstoff oder Halogen bedeutet und $R_3$ Wasserstoff oder niederes Alkyl bedeutet und n 2-6 ist.

2. Die Verbindung gemäss Anspruch 1, worin $R_1$ Halogen bedeutet.

3. Die Verbindung gemäss Anspruch 2, worin $R_2$ Wasserstoff bedeutet.

4. Die Verbindung gemäss Anspruch 3, worin $R_3$ Wasserstoff bedeutet.

5. Die Verbindung gemäss Anspruch 4, worin n 2 ist.

6. Die Verbindung gemäss Anspruch 5, worin $R_1$ Chlor bedeutet, wobei diese Verbindung die Formel

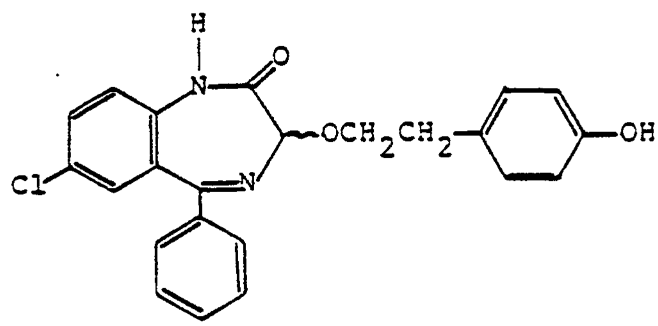

I(a)

aufweist.

7. Die Verbindung gemäss Anspruch 1 in radioaktiv markierter Form.

8. Die Verbindung nach Anspruch 7, welche mit $J^{125}$ radioaktiv markiert ist.

9. Die Verbindung nach Anspruch 8, welche mit $J^{125}$ markiert ist und $J^{125}$-7-Chlor-3-[2-(4-hydroxyphenyl)-äthoxy]-5-phenyl-1,3-dihydro-2H-1,4-benzodiazepin-2-on ist.

10. Die Verbindung gemäss Anspruch 1 in radioaktiv markierter Form.

11. Verwendung einer radioaktiv markierten Verbindung der Formel

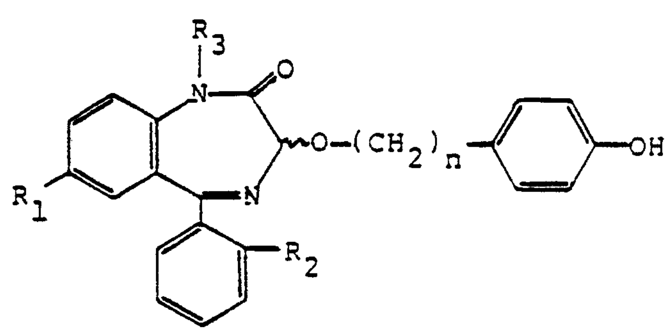

I

worin $R_1$ Wasserstoff oder Nitro bedeutet, $R_2$ Wasserstoff oder Halogen bedeutet und $R_3$ Wasserstoff oder niederes Alkyl bedeutet und n 2-6 ist, in einem Radioimmunverfahren zum Auffinden der Gegenwart von Benzodiazepinen.